# EUROPEAN PATENT SPECIFICATION

(11) **EP 1 429 845 B1**
(45) Date of publication and mention of the grant of the patent: **11.03.2009**
(21) Application number: 02754637.3
(22) Date of filing: 07.06.2002
(51) Int. Cl.: A61P 37/06, A61P 3/10, A61K 45/06, A61K 31/66

(54) **TREATMENT OR PROPHYLAXIS OF INSULIN-PRODUCING CELL GRAFT REJECTION**
BEHANDLUNG UND VORBEUGUNG VON INSULIN-PRODUZIERENDE ZELLTRANSPLANTATABSTOSSUNG
TRAITEMENT OU PROPHYLAXIE DU REJET DES GREFFES DE CELLULES PRODUISANT DE L'INSULINE

(30) Priority: 08.06.2001 US 297069 P
(43) Date of publication of application: 23.06.2004
(73) Proprietor: NOVARTIS AG, 4056 Basel (CH); Novartis Pharma GmbH, 1230 Wien (AT)
(72) Inventor: LAKE, Philip, Morris Plains, NJ 07950 (US); MULLON, Claudy, Acton, MA 01720 (US)
(74) Representative: de Weerd, Petrus G.W.
(86) International application number: PCT/EP2002/006278
(87) International publication number: WO 2002/100148

(56) References cited:
- WO-A-02/067915
- US-A- 5 952 316
- US-A- 6 004 565
- SHAPIRO A M ET AL: "Islet transplantation in seven patients with type 1 diabetes mellitus using a glucocorticoid-free immunosuppressive regimen." THE NEW ENGLAND JOURNAL OF MEDICINE. UNITED STATES 27 JUL 2000, vol. 343, no. 4, 27 July 2000 (2000-07-27), pages 230-238, XP002220559 ISSN: 0028-4793
- WHITE S A ET AL: "Human islet cell transplantation--future prospects." DIABETIC MEDICINE: A JOURNAL OF THE BRITISH DIABETIC ASSOCIATION. ENGLAND FEB 2001, vol. 18, no. 2, February 2001 (2001-02), pages 78-103, XP002220560 ISSN: 0742-3071
- DATABASE BIOSIS [Online] BIOSCIENCES INFORMATION SERVICE, PHILADELPHIA, PA, US; July 1998 (1998-07) YAMASAKI TORU ET AL: "Effect of a new immunosuppressive agent, FTY720, on survival of islet allografts." Database accession no. PREV199800396576 XP002220561 & CELL TRANSPLANTATION, vol. 7, no. 4, July 1998 (1998-07), pages 403-406, ISSN: 0963-6897

## Description

The present invention relates to a method of treatment or prophylaxis of insulin-producing cell graft rejection, particularly pancreatic islet graft rejection.

Type 1 diabetes is caused by a progressive, autoimmune destruction of the insulin-producing β-cells within the islets of the pancreas. At present, multiple daily insulin injections, or insulin pump therapy, remain the treatments of choice for the majority of diabetic patients. Intensive insulin therapy can decrease the incidence of secondary complications, but the effect is not absolute and patients are at increased risk for serious episodes of hypoglycemia.

Islet transplantation is a significantly safer method for replacing the diseased glandular tissue in diabetics than pancreatic organ transplantation, and has been investigated for more than 10 years as a treatment for type 1 diabetes mellitus in patients with inadequate glucose control despite intensive insulin therapy.

The majority of islet transplant procedures have been performed in kidney graft recipients already receiving an immunosuppressive regimen consisting of antibody induction with antilymphocyte globulin and life-long treatment with ciclosporine, azathioprine and glucocorticoids, see Brendel et al., International Islet Transplant Registry report., Giessen, Germany, 1999, pp. 1-20.

However, islet engraftment has been difficult to achieve with such an immunosuppressive regimen due to rejection, recurrent autoimmunity, primary nonfunction (PNF), and the diabetogenicity of conventional immunosuppressive drugs. In particular, proinflammatory mediators, produced by activated intrahepatic macrophages and endothelial cells subsequent to islet infusion, are detrimental to islet function and may lead to early islet loss or primary nonfunction of the graft.

Thus it has been estimated that over 10,000 islet equivalents (IEQ) per kg of recipient body weight are required in order to reproducibly achieve insulin independence in non-human primates (baboons, rhesus and cynomolgus monkeys), see Kenyon et al, Diabetes, 48, pp. 8132-8137,1999 and humans, see Shapiro et al, The New England J. of Med., 343 (4), pp. 230-238,2000.

Yamasaki and co-workers have reported achieving prolonged islet allograft survival of up to 20 days in male rats rendered hyperglycemic with streptozotocin by pre-administration of FTY720 the day before and the day of grafting, Cell Transplantation, Vol. 7, No. 4, pp. 403-406(1998).

Kenyon and co-workers demonstrated that islet transplantation can result in the reversal of hyperglycemia and in long-term insulin independence in humans and in several animal models of diabetes, including rodents, dogs, cynomolgus monkeys, rhesus monkeys, and baboons (Kenyon et al., Diabetes, 48, pp. 8132-8137, 1999), using an immunosuppresive regimen consisting of anti-CD154.

Shapiro and co-workers recently reported achieving favorable results in patients with type 1 diabetes and a history of severe hypoglycemia and metabolic instability who underwent islet transplantation in conjunction with a glucocorticoid-free immuno- suppressive regimen consisting of rapamycin (i.e. sirolimus), tacrolimus and daclizumab (i.e. a humanized antibody to the IL-2 receptor). Seven out of seven patients who received islet allografts became insulin independent. The longest reported patient follow up was 17 months posttransplantation, see Shapiro et al., NEJM 343:230-238 (2000).

US6004565A describes the use of FTY720 for the treatment or prevention of rejection of transplanted pancreatic islet cells. US5952316A describes a pharmaceutical composition comprising FTY720 and tacrolimus.W021067915 discloses the use of FTY720 and everolimus in the prevention of graft rejection.

Combination of FTY720 and rapamycin or everolimus for the treatment of pancreatic transplantation have been described, for example in EP1312359A2.

In presenting the future prospects in human islet cell transplantation, White and co-workers (Diabetic Medecine: A Journal of the British Diabetic Association, Vol. 18, No.2, p78-103), discussed the reasons why insulin-dependence after islet transplantation has been difficult to achieve, even with drugs which have been shown to have good immunosuppressive activity.

However, there is still a need for an improved therapy to achieve improved insulin-producing cell engraftment, e.g. pancreatic islet engraftment with an improved quality of life.

It has now been found that co-administration of the accelerated lymphocyte homing ("ALH") agent 2-amino-2-[2(4-octylphenyl)ethyl]propane-1,3-diol with one or more immunosuppressive agents acting via a different mechanism than the ALH agent, to an islet graft recipient, provides an effective treatment or prophylaxis of pancreatic islet cell transplant rejection, and in particular enables type I diabetic transplant patients to achieve extended insulin independence.

In a particular embodiment the present invention comprises a method for the treatment or prophylaxis of insulin-producing cell graft rejection in an insulin-producing cell graft recipient comprising co-administering to the recipient an effective amount of 2-amino-2-[2-(4-octylphenyl)ethyl]propane-1,3-diol, the antibody to the IL-2 receptor basiliximab, and the immuno suppressive macrocyclic lactone everolimus, and optionally a soluble human complement inhibitor. Preferably the co-administration therapy of the invention is glucocorticoid-free.

The combination therapy of the invention facilitates engraftment, sustained insulin independence, and long-term survival of insulin-producing cell allo- or xenografts. A particular advantage of the present therapy is in facilitating single-donor transplants, which are less clinically challenging than multiple donor grafts, by effectively reducing the numbers of transplanted cells needed to provide functional insulin-producing cell mass in the patient. For example, the present therapy can reduce the required number of islet equivalents (IEQ) to 5,000 mg/kg per recipient, or less.

By "insulin independence" is meant endogenous insulin production as determined after intravenous glucose tolerance test to the extent that the subject has normal glucose tolerance.

By "insulin-producing cell" are meant islets of Langerhans (of allo or xeno origin) and other cells such as suitable insulin-secreting cells or cell lines, e.g. stem cell derived or cloned insulin-secreting cells.

As alternative to the above, the present invention also provides:
1. Use of the ALH agent of the invention in free form or in pharmaceutically acceptable salt form in combination with basiliximab and everolimus and optionally a soluble human complement inhibitor,
   to treat or prevent insulin-producing cell graft rejection.
2. A pharmaceutical combination comprising a) the ALH agent of the invention in free form or in pharmaceutically acceptable salt form, b) the antibody to the IL-2 receptor basiliximab, and c) the immunosuppressive macrocyclic lactone everolimus and optionally a soluble human complement inhibitor.
   The term "pharmaceutical combination" as used herein preferably includes a non-fixed combination, e.g. the active components are administered to a patient as separate entities either simultaneously, concurrently or sequentially with no specific limits, wherein such administration provides therapeutically effective levels of the components in the body of the patient Each active component may be administered in the form of a pharmaceutical composition, e.g. the active component is associated with one or more pharmaceutically acceptable diluents or carriers therefor. ,
3. Use of a pharmaceutical combination as described above in a method as disclosed above.
4. Use of the ALH agent of the invention in free form or in pharmaceutically acceptable salt form, in the manufacture of a medicament for use in treating or preventing insulin-producing cell graft rejection in an insulin-producing cell graft recipient, in combination with the antibody to the IL-2 receptor, basiliximab, and the immunosuppressive macrocyclic lactone everolimus and optionally a soluble human complement inhibitor.

The ALH agent of the invention is FTY720, i.e. 2-amino-2-[2-(4-octylphenyl) ethynpropane-1,3-diol in free form or in a pharmaceutically salt form, e.g. the hydrochloride, as shown: 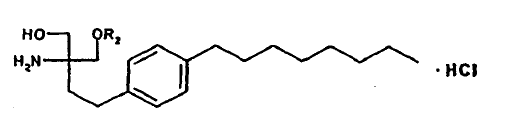 wherein R₂ is H,
or its corresponding phosphate, wherein R₂ is 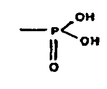 in free form or in a pharmaceutically acceptable salt form.

A disclosure of compounds, substituent groups, and variations included in the ALH-compound of this invention and methods of preparing said compounds can be found in U.S. Patent Nos. 5,604229 and 6,004,565, incorporated herein by reference in their entirety.

Examples of pharmaceutically acceptable salts include salts with inorganic acids, such as hydrochloride, hydrobromide and sulfate, salts with organic acids, such as acetate, fumarate, maleate, benzoate, citrate, malate, methanesutfonate and benzenesulfonate salts, or, when appropriate, salts with metals such as sodium, potassium, calcium and aluminium, salts with amines, such as triethylamine and salts with dibasic amino acids, such as lysine. The compounds and salts of the methods of the present invention encompass hydrate and solvate forms.

FTY720, a novel immunomodulator, increases the responsiveness of lymphocytes to homing chemokines. Naïve cells are sequestered; CD4 and CD8 T-cells and B-cells from the blood are stimulated to migrate into lymph nodes (LN) and Peyer's patches (PP), and infiltration of cells into transplanted organs is inhibited. However, FTY720 does not impair lymphocyte activation, expansion and memory within the lymphoid system, and therefore does not suppress immunity to systemic infection.

The anti-IL-2 receptor antibody of the invention is basiliximab (Simulect^{™}), which is a chimeric antibody comprising the variable region of murine monoclonal antibody CHI-621 and a human IgG1 region, see EP 449,769, incorporated herein by reference.

By "immunosuppressive macrocyclic lactone" is meant e.g. 40-O-(2-hydroxyethyl) rapamycin, i.e. everolimus.

Suitable soluble complement inhibitor includes e.g. a C3/C5 inhibitor, e.g. a soluble complement receptor type I (CR1), TP-10, which is a recombinant protein that is a potent systemic inhibitor of the complement system, since it blocks both C3 and C5 activation by all three activation pathways (classical, alternative, and lectin); and it is subsequent to C3 activation that the majority of complement-dependent effector mechanisms are recruited. Specifically, TP-10 binds C3b and C4b, activation fragments of the complement system, blocking their interaction with other proteins in the complement cascade and subsequently the formation of multi-molecular enzyme complexes which generate the biologically active protein fragments of complement. Top-10 also acts as a co-factor in the enzymatic degradation of C3b and C4b to their inactive forms.

TP-10 is a modified CR1 molecule lacking the transmembrane and cytoplasmic domains, e.g. as disclosed in WO 89/09220, incorporated herein by reference. TP-10 is expressed by Chinese hamster ovary (CHO) cells in serum-free media and purified on anti-CR1 affinity columns and by HPLC. Administration of TP10 to islet transplant recipients was reported by Bennett, et. al., Diabetes 2000.

Other embodiments of a soluble complement receptor inhibitor suitable for use in the invention comprise TP-20, a combined complement and selectin inhibitor that integrates sCR1 (soluble complement receptor-1) with the sLex (sialyl Lewis x) carbohydrate in a single molecule; and Top-18, an sCR1 derivative inserted into a selectin-(receptor)-blocking carbohydrate.

Daily dosages of the therapeutic agents required in practicing the method of the present invention will vary, depending upon for example the host, the mode of administration, the severity of the condition to be treated, and the further selected therapeutic agents used in combined administration.

The terms "co-administration" or "combined administration" or the like as utilized herein are meant to encompass administration of the selected therapeutic agents to a single transplant recipient, and are intended to include treatment regimens in which the agents are not necessarily administered by the same route of administration or at the same time.

It is preferred that administration of the ALH agents, i.e., FTY720, be commenced preoperatively. In general, the compound may be administered starting from just prior to the day the transplant operation is carried out (i.e. "Day 0"), for example starting on Day -1, and continuing indefinitely thereafter. The compound may be administered e.g. orally or by injection.

A preferred daily dosage range for the ALH agent, i.e., FTY720, is about from 0.03 to 2.5 mg/kg per day, particularly 0.1 to 2.5 mg/kg per day, e.g. 0.5 to 2.5 mg/kg per day as a single dose or in divided doses. Suitable daily dosages for patients are in the order of from e.g. 0.25 to 100 mg p.o. As an alternative, FTY720 in free form or in pharmaceutically acceptable salt form may also be administered twice or three times a week, e.g. at a dosage as indicated above. The ALH agent, i.e FTY720, may be administered by any conventional route, in particular enterally, e.g. orally, for example in the form of solutions for drinking, tablets or capsules or parenterally, for example in the form of injectable solutions or suspensions. Pharmaceutical compositions comprising the compounds of formula I may be manufactured in conventional manner, e.g. as described in U.S. Patent No. 5,604,229.

The anti-IL2 receptor antibody, i.e., basiliximab, is preferably administered in a two-dose regimen, the first dose being administered on Day 0 (i.e. day of transplant) and a second on about Day 4. Additionally doses following about Day 4 may optionally be administered, e.g. once weekly for 2 to 4 weeks. For primates, including humans, each dose is generally about 1 to 50 mg, and preferably about 5 to 20 mg.

The immunosuppressive macrocyclic lactone, i.e., 40-O-2-(hydroxyethyl)-rapamycin, is preferably administered on a daily basis, commencing on or just prior to the day of transplant (e.g., Day -1) and continuing on an indefinite basis following the transplant For primates, including humans and non-human primates, suitable doses are in the range of 0.25 - 7 mg /day, and more particularly 0.5 - 5 mg /day. The compound may be administered orally or alternatively by subcutaneous injection.

The soluble complement receptor, e.g., TP-10, can be administered in single dosages of about 5 to 15 mg/kg, preferably about 10 mg/kg, as an intravenous infusion over about 30 minutes.

Most preferably, the invention is directed to a glucocorticoid-free combination therapy for use in connection with insulin-producing cell transplantation, e.g. pancreatic islet cell transplantation, comprising co-administration of 2-amino-2-[2-(4-octylphenyl)ethyl]propane-1,3-diol in free form or in a pharmaceutically acceptable salt form, preferably the hydrochloride salt thereof; in combination with basiliximab, 40-O-(2-hydroxyethyl)-rapamycin and optionally the soluble recombinant human complement inhibitor, sCR1 ("TP10").

The therapeutic methods of the invention may optionally include co-administration of still other immunomodulating drugs or anti-inflammatory agents, examples of which may comprise a calcineurin inhibitor, e.g. cyclosporins or ascomycins, and their immunosuppressive analogs, e.g. cyclosporin A, FK-506; cyclophosphamide; azathioprene; methotrexate; brequinar; leflunomide; mizoribine; mycophenolic acid; mycophenolate mofetil; 15-deoxyspergualine or analogues; immunosuppressive monoclonal antibodies, e.g., monoclonal antibodies to leukocyte receptors, e.g., to MHC, CD2, CD3, CD4, CD7, CD25, CD28, B7, CD40, CD45, or CD58 or to their ligands; or other immunomodulatory compounds, e.g. CTLA4-lg or a homolog or mutant thereof, e.g. LEA29Y, or a LFA-1 inhibitor.

The methods of the invention may be employed as a prophylaxis or treatment of insulin-producing cell allograft or xenograft rejection.

### Example: Transplantation of allogeneic islets into cynomolgus monkeys suppressed with FTY 720, Everolimus, Basiliximab and TP10.

### Therapeutic agents:

FTY720: The compound is prepared for administration by emptying the contents of a capsule (1 mg/capsule) in a 60 mL clear glass mortar. 30 mL of sterile water are added and mixed with the capsule content until the powder is in a uniform suspension. The FTY720 is administered orally using a syringe and a nasogasitric tube.

Basiliximab: The material is obtained as a package containing 20 mg of powder in a vial and a second vial containing 5 mL of diluent. Each vial is formulated according to the manufacturer's instructions and administered i.v. accordingly.

Everolimus: The compound is obtained as a concentrate of 20 mg/ml in a sealed ampoule. 1 ml of the concentrate is mixed with 8.5 ml vehicle (50% Cremophor and 50% ethanol) to give a final concentration of 2.1 mg/ml (pH 6.0) and the mixture is used within 2 hours.

TP10: the material is obtained as 50 mg multi-dose vials (no formulation required).
(a) Pancreatectomy of donor animals Donor animals are adult cynomolgus monkeys over 4 kg, of either sex. Prior to pancreatectomy, fasting serum glucose analysis and aginine stimulation are performed to assure normal endocrine pancreatic function. For arginine stimulation, 0.07 mg/kg arginine are injected intravenously, and blood collected at -5, -1, 2, 3, 4, and 5 minutes after arginine injection. Plasma is collected and stored frozen at -80°C. Plasma insulin and C-peptide levels are confirmed to be within normal range.
   Pancreatectomy is performed under general anaesthesia with 0.5 to 2% isofluorane, and the pancreas is harvested. Lymph nodes and spleen are also harvested for donor lymphocyte isolation and cryopreservation. Following organ and tissue harvest the donor is euthanized with 150 mg/Kg of Na pentobarbital i.v., and cardiac arrest confirmed by visual inspection.
(b) Islet isolation Islet isolation is performed using modifications of the automated method for human islet isolation, as described by Ricordi et al., Diabetes, 38, (Suppl.1): 140-142, 1989; Kenyon et al., Diabetes, 48, pp. 8132-8137,1999; and Ranuncoli et al., Cell Transplantation 2000.

Islet quality assessment is performed according to international standards {Ricordi, Gray, et al. 1990 13/id}, including determination of islet yield, purity, and viability. The number of islets obtained is reported as islet equivalents (IEQ), which is the number of islets that would be present if the particles were all 150 µm in diameter. For this purpose, the number of dithizone (DTZ) positive islets in different size categories (50-100 µm, 100-150 µm, 150-200 µm, etc.) is counted and the data will be entered into a computer program that translates the information into IEQ. Purity is estimated based on the percentage of DTZ positive particles present in the preparation, and viability is estimated based on FDA/PI staining. In vitro functional capacity is determined via assessment of glucose stimulated insulin release in static cultures (see Ranuncoli et. al., Cell Transplantation, 2000).

(b) Transplant of islet allografts into recipient animals Recipient animals are juvenile cynomolgus monkeys of > 1.5 kg, of either sex. Recipient animals are rendered diabetic by infusion of streptozotocin (STZ), 150 mg/kg i.v., followed by intravenous hydration (20 ml/kg of 0.9% NaCl over 30 minutes) to prevent nephrotoxic side effects, as described by Theriault Thistlethwaite et al., 1999, 16 /id. Blood glucose level is monitored frequently during the first 48 hours after streptozotocin application to avoid severe hypoglycemia or hyperglycemia with eventual ketoacidosis. Thereafter, blood sugar levels are monitored 2-3 times daily and corrected with Regular, NPH, Lente, Ultralente, or Humalog insulin via subcutaneous injection or intravenous insulin-drip, as needed. Induction of diabetes is confirmed by daily blood glucose measurements, assessment of insulin requirements and by a negative C-peptide value subsequent to arginine stimulation. To confirm that diabetes has been induced, an arginine stimulation test is performed prior to initiation of immunosuppression. For arginine stimulation, 0.07 mg/kg arginine is injected intravenously, and blood collected - 5, -1, 2, 3, 4, 5 min. after arginine injection. Plasma is collected and stored frozen at -80°C. Plasma glucose is measured using a Cobas Mira glucose analyzer (Roche Diagnostic Systems, Montclair, NJ). A double antibody method (Diagnostic Products, Corp., Los Angeles, CA) is utilized to assess plasma insulin and C-peptide levels. The lower limit of detection for C-peptide is 0.20 ng/ml and for insulin is 5 uU/ml. Standard curves, as well as positive and negative control samples are incorporated into the assays.

Sedated and anaesthetized recipient animals are placed supine on the operating table and the abdomen is prepped and draped. Special attention is paid to avoid hypothermia and a heat lamp and heating pads are used. A small central midline incision is made and a minimum of 10,000 freshly isolated islet equivalents/kg body weight are resuspended in 20 ml of transplant media and infused into a mesenteric tributary of the portal vein through a 24-gauge intravenous catheter. To provide hemostasis, the vessel is either ligated or digital pressure is applied. The abdominal wall is closed in a routine fashion. Blood glucose is monitored twice each day and arginine stimulation tests are performed to document reversal of the diabetic state.

Three groups of 4 recipients each are administered different immunosuppressive regimens and islet doses, as follows:
Group 1: FTY720 + everolimus + basiliximab, 10,000 IEQ/kg body weight.
Group 2: FTY720 + everolimus + basiliximab, 5,000 IEQ/kg body weight.
Group 3: FTY720 + everolimus + basiliximab + TP10, 5,000 IEQ/kg body weight.

Recipient animals in the indicated groups receive the following treatments prior to and following the day of transplant (Day "0") as detailed below:
FTY720 is administered p.o at 0.3 mg/kg, day-1 through day +30.

Basiliximab is administered by intravenous injection at 10 mg on day 0 and day 4. Everolimus administered by subcutaneous injection once daily from day -1 through day +30, targeting trough levels of 15-30 ng/ml. The recommended dose is in the range of 0.075 mg/kg/day.

TP10 is administered by intravenous injection at a dosage level of 40 mg/kg on day -1, 20 mg/kg on days 0 and 1, and 17 mg/kg from days 2 to 7.

Drug monitoring is as follows:
FTY720 - weekly. Everolimus - twice weekly. SC5b-9 is monitored for proof of TP10 efficacy.

The recipient animals are dosed at approximately the same time each morning and in the afternoon when applicable. Blood glucose levels are determined frequently over the first 4-5 hours post-transplant to prevent hypoglycemic episodes. Such episodes are treated with dextrose 5-10% intravenously as needed. The presence of rejection is suspected if three consecutive fasting blood glucose levels rise above 150 mg/dl or three post-prandial blood glucose levels of more than 200 mg/dl are recorded. Rejection is assumed if these levels are present 3 days in a row. Thereafter, fasting and post-prandial blood glucose (fasting blood glucose, post-prandial glucose) levels are monitored 2-3 times a day via heel stick. Daily blood glucose measurements plus periodic arginine stimulation tests (human insulin and C-peptide) at 14 and 30 days posttransplant are used to document glucose control. Exogenous insulin requirement after transplantation is also monitored. For the first 14 days after transplantation, blood glucose levels are corrected with Regular, NPH, Lente, or Ultralente Humalog insulin via subcutaneous injection according to an individualized sliding scheme. An arginine stimulation test is performed at 14 days and 1 month posttransplant and at additional time points thereafter.

(c) Pre-terminal blood and tissue sampling The transplant recipients are sedated by ketamine (5-10 mg/kg), intubated and pre-terminal blood sampling is performed under general anaesthesia with 0.5 to 2% isofluorane. The monkey is put in a supine position and the groin and abdomen are prepped and draped in a sterile fashion. The femoral vein, superior vena cava, or aorta is isolated, a catheter is placed, and 80-160 ml of blood are drawn. Samples of liver and spleen are harvested and frozen in liquid Nitrogen for RNA analysis. After completion of sterile sampling, the animal is euthanized by i.v. injection of sodium pentobarbital at a dose of 150 mg/Kg. Necropsy samples are taken and fixed according to known procedures. Besides graft and organ samples, a sample of the pancreas is snap-frozen for insulin extraction.

The pathology of the graft organ is evaluated. Routine H&E stained sections are evaluated histologically. In this model, the combined treatment with FTY720, everolimus and basiliximab prevents islet allograft rejection.

## Claims

1. A pharmaceutical combination comprising a) 2-amino-2-[2-(4-octylphenyl)ethyl]propane-1,3-diol in free form or in a pharmaceutically acceptable salt form; b) basiliximab, and c) 40-O-(2-hydroxyethyl)-rapamycin.

2. A combination according to claim 1 for use in the treatment or prophylaxis of insulin-producing cell graft rejection.

3. A combination according to claim 1 or claim 2 for the preparation of a medicament to provide functional insulin-producing cell mass in a recipient transplanted with 5,000 IEQs per Kg of recipient bodyweight or less.

4. Use of a pharmaceutical combination comprising a) 2-amino-2-[2-(4-octylphenyl)ethyl]propane-1,3-diol in free form or in a pharmaceutically acceptable salt form; b) basiliximab and c) 40-O-(2-hydroxyethyl)-rapamycin, for the preparation of a medicament for the treatment or prophylaxis of insulin-producing cell graft rejection.

5. A combination according to claim 1 further comprising a soluble human complement inhibitor.

6. Use of a pharmaceutical combination according to claim 5, for the preparation of a medicament for the treatment or prophylaxis of insulin-producing cell graft rejection.

7. A combination according to any one of claims 1, 2, 3 or 5 wherein 2-amino-2-[2-(4-octylphenyl)ethyl]propane-1,3-diol is in free form of hydrochloride.

8. An use according to claim 4 or 6 wherein 2-amino-2-[2-(4-octylphenyl)ethyl]propane-1,3-diol is in free form of hydrochloride.

## Patentansprüche

1. Pharmazeutische Kombination, umfassend a) 2-Amino-2-[2-(4-octylphenyl)-ethyl]-propan-1,3-diol in freier Form oder in Form eines pharmazeutisch akzeptablen Salzes, b) Basiliximab und c) 40-0-(2-Hydroxyethyl)-rapamycin.

2. Kombination nach Anspruch 1 zur Verwendung für die Behandlung oder Prophylaxe einer Zellpfropfabstoßung.

3. Kombination nach Anspruch 1 oder 2 zur Herstellung eines Arzneimittels zur Bildung einer funktionalen Insulin-produzierenden Zellmasse bei einem Patienten, in den 5000 IEQs pro kg des Körpergewichts des Rezipienten oder weniger transplantiert sind.

4. Verwendung einer pharmazeutischen Kombination, umfassend a) 2-Amino-2-[2-(4-octylphenyl)-ethyl]-propan-1,3-diol in freier Form oder in Form eines pharmazeutisch akzeptablen Salzes, b) Basiliximab und c) 40-O-(2-Hydroxyethyl)-rapamycin, zur Herstellung eines Arzneimittels für die Behandlung oder Prophylaxe einer Insulin-produzierenden Zellpfropfabstoßung.

5. Kombination nach Anspruch 1, weiter umfassend einen löslichen humanen Komplementinhibitor.

6. Verwendung einer pharmazeutischen Kombination nach Anspruch 5 zur Herstellung eines Arzneimittels für die Behandlung oder Prophylaxe einer Insulin-produzierenden Zellpfropfabstoßung.

7. Kombination nach einem der Anspruche 1, 2, 3 oder 5, worin das 2-Amino-2-[2-(4-octylphenyl)-ethyl]-propan-1,3-diol in freier Form eines Hydrochlorids vorliegt.

8. Verwendung nach Anspruch 4 oder 6, worin das 2-Amino-2-[2-(4-octylphenyl)-ethyl]-propan-1,3-diol in freier Form eines Hydrochlorids vorliegt.

## Revendications

1. Combinaison pharmaceutique comprenant a) du 2-amino-2-[2-(4-octylphényl)éthyl]propane-1,3-diol sous forme libre ou sous forme d'un sel pharmaceutiquement acceptable ; b) du basiliximab, et c) de la 40-O-(2-hydroxyéthyl)rapamycine.

2. Combinaison selon la revendication 1, à utiliser dans le traitement ou la prophylaxie d'un rejet de greffe de cellules productrices d'insuline.

3. Combinaison selon la revendication 1 ou 2, pour la préparation d'un médicament pour fournir une masse fonctionnelle de cellules productrices d'insuline chez un receveur ayant subi une transplantation de 5000 IEQ par kg de poids corporel du receveur ou moins.

4. Utilisation d'une combinaison pharmaceutique comprenant a) du 2-amino-2-[2-(4-octylphényl)éthyl]propane-1,3-diol sous forme libre ou sous forme d'un sel pharmaceutiquement acceptable ; b) du basiliximab, et c) de la 40-O-(2-hydroxyéthyl)rapamycine, pour la préparation d'un médicament pour le traitement ou la prophylaxie d'un rejet de greffe de cellules productrices d'insuline.

5. Combinaison selon la revendication 1, comprenant en outre un inhibiteur de complément humain soluble.

6. Utilisation d'une combinaison pharmaceutique selon la revendication 5, pour la préparation d'un médicament pour le traitement ou la prophylaxie d'un rejet de greffe de cellules productrices d'insuline.

7. Combinaison selon l'une quelconque des revendications 1, 2, 3 et 5, dans laquelle le 2-amino-2-[2-(4-octylphényl)éthyl]propane-1,3-diol est sous forme libre de chlorhydrate.

8. Utilisation selon la revendication 4 ou 6, dans laquelle le 2-amino-2-[2-(4-octylphényl)éthyl]propane-1,3-diol est sous forme libre de chlorhydrate.
